(19) Europäisches Patentamt
European Patent Office
Office européen des brevets

(11) EP 1 848 439 B1

(12) **EUROPEAN PATENT SPECIFICATION**

(45) Date of publication and mention of the grant of the patent:
**10.03.2010 Bulletin 2010/10**

(21) Application number: **05821493.3**

(22) Date of filing: **20.12.2005**

(51) Int Cl.:
***A61K 31/55*** *(2006.01)* ***A61K 47/26*** *(2006.01)*
***A61K 47/34*** *(2006.01)* ***A61K 47/02*** *(2006.01)*

(86) International application number:
**PCT/EP2005/056970**

(87) International publication number:
**WO 2006/067150 (29.06.2006 Gazette 2006/26)**

(54) **ORAL IMMEDIATE RELEASE FORMULATION OF A POORLY WATERSOLUBLE ACTIVE SUBSTANCE**

ORAL ANZUWENDENDE SCHNELLFREISETZENDE ARZNEIMITTEL SCHWER LÖSLICHER WIRKSTOFFE

COMPOSITIONS PHARMACEUTIQUES DE COMPOSÉS ACTIFS DIFFICILEMENT SOLUBLES À LIBÉRATION IMMÉDIATE ET POUR ADMINISTRATION ORALE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **23.12.2004 US 638111 P**
**23.12.2004 EP 04106933**

(43) Date of publication of application:
**31.10.2007 Bulletin 2007/44**

(73) Proprietors:
- **Solvay Pharmaceuticals B.V.**
  **1381 CP Weesp (NL)**
- **Panacea Biotec Ltd.**
  **New Delhi 110 044 (IN)**

(72) Inventors:
- **SINGH, Sukhjeet c/o Panacea Biotec Ltd.**
  **Lalru, Punjab 140 501 (IN)**
- **JAIN, Rajesh c/o Panacea Biotec Ltd. B-1**
  **New Delhi 110 044 (IN)**
- **KUMAR, Rohit c/o Panacea Biotec Ltd.**
  **Lalru, Punjab 140 501 (IN)**
- **DE WINTER, Marius L. c/o Solvay Pharmaceuticals**
  **NL-1381 CP Weesp (NL)**

(74) Representative: **Verhage, Marinus et al**
**Octrooibureau Zoan B.V.**
**P.O. Box 140**
**1380 AC Weesp (NL)**

(56) References cited:
**EP-A- 0 733 642** **EP-A- 0 830 863**
**WO-A-03/068266** **WO-A-2004/062692**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 848 439 B1

## Description

The present invention relates to an oral immediate release formulation of an active compound of the general formula

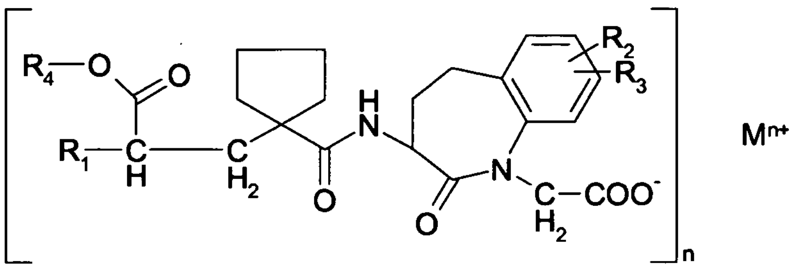

(I)

wherein:

$R_1$ is a selected from the group consisting of $(C_1-C_6)$alkoxy$(C_1-C_6)$alkyl which may be substituted by a $(C_1-C_6)$ alkoxy, phenyl-$(C_1-C_6)$-alkyl and phenyloxy-$(C_1-C_6)$-alkyl wherein the phenylgroup may be substituted with $(C_1-C_6)$ alkyl, $(C_1-C_6)$alkoxy or halogen, and naphtyl-$(C_1-C_6)$-alkyl,
$R_2$ and $R_3$ are both independently hydrogen or halogen,
$R_4$ is a biolabile ester forming group,
M is a hydrogen or a metal ion, preferably a bivalent metal ion.
n is 1, 2 or 3;

Various active substances, including the compounds of formula (I) mentioned above have a very poor solubility in water. When these active substances are administered to the body, they often have a poor bio-availability due to the poor solubility in the digestive fluid. In order to solve this problem several methods were developed, such as micronization, inclusion in cyclodextrins, the use of inert watersoluble carriers, the use of solid dispersions (WO 00/00179) or solid solutions or nanocrystalline or amorphous forms of an active substance.
WO 03/068266 describes an oral solid solution formulation of compounds of formula (I) having enhanced bio-availability compared with said active substance in a traditionally formulated form. Although this formulation has superior bioavailability properties, it has the draw-back that it is formed via a melt mixture leading to some restrictions: it has to be formulated either into a capsule, or into a tablet via melt-extrusion technique. Further the size of the formulation will be too large for higher dosages.
It is the objective of the present invention to provide an alternative oral formulation for the compound of formula I as defined above with a significant increase in bio-availability compared with said active substance in a traditionally formulated form that is sufficiently stable for commercial use and that also can be used to prepare formulations with a high content of active substance with a reasonable size. It is a further objective of the present invention to provide a formulation which can be prepared using normal formulation procedures and equipment, so that no large investments are necessary.
This objective can be achieved, according to the present invention, by a n oral immediate release formulation of an active compound of the general formula

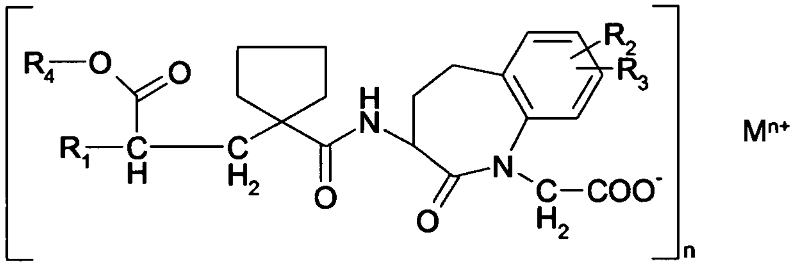

(I)

wherein:

$R_1$ is a selected from the group consisting of $(C_1-C_6)$alkoxy$(C_1-C_6)$alkyl which may be substituted by a $(C_1-C_6)$ alkoxy, phenyl-$(C_1-C_6)$-alkyl and phenyloxy-$(C_1-C_6)$-alkyl wherein the phenylgroup may be substituted with $(C_1-C_6)$ alkyl, $(C_1-C_6)$alkoxy or halogen, and naphtyl-$(C_1-C_6)$-alkyl,
$R_2$ and $R_3$ are both independently hydrogen or halogen,
$R_4$ is a biolabile ester forming group,
M is a hydrogen or a metal ion, preferably a bivalent metal ion.
n is 1, 2 or 3;

comprising

a) said active substance in an amount of up to 65% of the total weight of the formulation;
b) at least 10% w/w an alkaline compound or a mixture of alkaline compounds;
c) between 0.1 and 10% w/w of one or more surfactants, and
d) optionally comprises auxiliary materials in an amount of between 1% and 45% of the total weight of the formulation.

M is selected from the group consisting of Li+, $Ca^{2+}$, $Mg^{2+}$ and $Zn^{2+}$, and is preferably $Ca^{2+}$. $(C_1-C_6)$-alkyl is defined as a straight or branched alkyl group consisting of between 1 and 6 carbon atoms. $(C_1-C_6)$-alkoxy is defined as a straight or branched alkoxy group consisting of between 1 and 6 carbon atoms. $R_1$ is preferably phenylethyl, $R_2$ and $R_3$ are preferably hydrogen and $R_4$ is preferably ethyl.

Compounds of the general formula (I) are disclosed in EP0733642 and in WO 03/059939.

The preferred compound is the calcium salt of 1H-1-Benzazepine-1-acetic acid, 3-[[[1-[2-(ethoxycarbonyl)-4-phenylbutyl]cyclopentyl]carbonyl]amino]-2,3,4,5-tetrahydro-2-oxo-. The most preferred compound is said compound in its 3S,2'R form. This compound is referred to as Compound S-Ca, the corresponding acid (1H-1-Benzazepine-1-acetic acid, 3-[[[1-[2-(ethoxycarbonyl)-4-phenylbutyl]cyclopentyl]-carbonyl]amino]-2,3,4,5-tetrahydro-2-oxo-) is referred to as Compound S-H and the corresponding S-α-methylbenzylamine salt is referred to as Compound S-Mba.

The active substance of formula (I) is normally used in an amount between about 0.1 and 60% by weight, more preferably in an amount between 1 and 45% by weight and most preferably in an amount between about 10 and 45% by weight. The active substance may optionally be used in a micronized form.

The following definitions are provided to facilitate understanding of certain terms used within the framework of the present application.
Immediate release refers to a release of at least 75 % of the drug in a dissolved form from the dosage form within 90 minutes.
Sufficiently stable for commercial use means an acceptable chemical and physical stability during a storage period of at least on e year at ambient conditions, preferably at least 2 years, even more preferably at least 3 years and most preferred at least 5 years. An acceptable chemical stability means not more than 5% degradation of the active material during the storage period, preferably not more than 3% and most preferably not more than 1%. An acceptable physical stability means no significant change in appearance, no tablet disintegration during deblistering at the end of the storage period and not more than 20% change of the disintegration time. The physical stability is also only acceptable at a dissolution of at least 70% of the active ingredient within 60 minutes during the whole storage period. The term "micronized" refers to a particle size wherein, on a volume basis, more than 95% of the particles is smaller than 75 microns.

The alkaline compound is selected from the group consisting of inorganic and organic alkaline compounds, such as sodium bicarbonate, potassium bicarbonate, sodium carbonate, potassium carbonate, sodium citrate, tris buffer, triethanolamine, alkaline hydroxides such as sodium hydroxide, potassium hydroxide or magnesium hydroxide, alkaline

phosphates such as dipotassium hydrogen phosphate, and meglumine. Also mixtures of these alkaline compounds can be used. Preferred alkaline compounds are sodium bicarbonate, potassium bicarbonate, sodium carbonate, potassium carbonate and calcium carbonate. The most preferred alkaline compound is sodium bicarbonate.

The alkaline compound is normally used in an amount of at least 10% of the total weight of the formulation. In case a carbonate is used, it is preferably used in an amount of 50% of the total weight of the formulation, more preferably in an amount of at least 55% w/w and most preferred in an amount of at least 60% w/w.

The surfactant ingredient is preferably a hydrophilic surfactants and more preferably a hydrophilic surfactant selected from the group consisting of non-ionic hydrophilic surfactants and anionic hydrophilic surfactants. Examples of non-ionic hydrophilic surfactants are polyoxyethylene sorbitan esters, cremophores and poloxamers. Examples of anionic surfactants are sodium lauryl sarcosinate, docusate and pharmaceutically acceptable docusate salts. Also a mixture of these surfactants can be used. More preferred are polyoxyethylene sorbitan esters, sodium lauryl sarcosinate, docusate and pharmaceutically acceptable docusate salts. Even more preferred are docusate calcium, docusate sodium and docusate potassium. The most preferred surfactant ingredient is docusate sodium. Docusates are commercially available (e.g. from Sigma Aldrich).

Docusates are normally provided as cubes with a side of about 1 cm. The docusate can be added to the dry ingredient mixture after cryogenic milling (i.e. milling at low temperature e.g. after cooling with solid carbon dioxide or liquid nitrogen) or as a solution in e.g. dichloromethane, ethyl acetate or methyl *t*-butyl ether. Alternatively the docusate can be co-precipitated with the active ingredient from an organic solution comprising both the active ingredient and the docusate by adding an anti-solvent, such as hexane.

The surfactant is normally used in an amount of between 0.1 % and 10% of the total weight of the formulation, preferably in an amount of between 0.5 and 2.5%, more preferably in an amount of between 0.8 and 1.5% w/w and most preferred in an amount of approximately 1.0 % w/w.

The weight ratio between the surfactant and the active compound is preferably between 1 : 200 and 1 : 5, more preferably between 1 : 30 and 1 : 10 and most preferred about 1 : 15. The weight ratio between the active compound and the alkaline compound is preferably between 1 : 6 and 1 : 0.5, more preferably between 1 : 5 and 1 : 1.5 and most preferred about 1 : 4. The weight ratio between the surfactant and the alkaline compound is preferably between 1 : 2000 and 1 : 5, more preferably between 1 : 100 and 1 : 10 and most preferred about 1 : 60.

The formulation optionally comprises auxiliary materials at an amount of up to 45% of the total weight of the formulation and preferably between 1% and 45% of the total weight of the formulation. Examples of these auxiliary materials are

a) Binders such as acacia, alginic acid and salts thereof, cellulose derivatives, methylcellulose, hydroxyethyl cellulose, hydroxypropyl cellulose, magnesium aluminum silicate, polyethylene glycol, gums, polysaccharide acids, bentonites, hydroxypropyl methylcellulose, gelatin, polyvinylpyrrolidone, polyvinylpyrrolidone/vinyl acetate copolymer, crospovidone, povidone, polymethacrylates, hydroxypropylmethylcellulose, hydroxypropylcellulose, starch, pregelatinized starch, ethylcellulose, tragacanth, dextrin, microcrystalline cellulose, sucrose, or glucose, and the like.
b) Disintegration agents such as starches, pregelatinized corn starch, pregelatinized starch, celluloses, cross-linked carboxymethylcellulose, crospovidone, cross-linked polyvinylpyrrolidone, a calcium or a sodium alginate complex, clays, alginates, gums, or sodium starch glycolate, and any disintegration agents used in tablet preparations.
c) Filling agents such as lactose, calcium carbonate, calcium phosphate, dibasic calcium phosphate, calcium sulfate, microcrystalline cellulose, cellulose powder, dextrose, dextrates, dextran, starches, pregelatinized starch, sucrose, xylitol, lactitol, mannitol, sorbitol, sodium chloride, polyethylene glycol, and the like.
d) Stabilizers such as any antioxidation agents, buffers, or acids, and the like.
e) Lubricants such as magnesium stearate, calcium hydroxide, talc, colloidal silicon dioxide, sodium stearyl fumarate, hydrogenated vegetable oil, stearic acid, glyceryl behenate, magnesium, calcium and sodium stearates, stearic acid, talc, waxes, Stearowet, boric acid, sodium benzoate, sodium acetate, sodium chloride, DL-leucine, polyethylene glycols, sodium oleate, or sodium lauryl sulfate, and the like.
f) Wetting agents such as oleic acid, glyceryl monostearate, sorbitan monooleate, sorbitan monolaurate, triethanolamine oleate, pol yoxyethylene sorbitan monooleate, polyoxyethylene sorbitan monolaurate, sodium oleate, or sodium lauryl sulfate, and the like.
g) Diluents such lactose, starch, mannitol, sorbitol, dextrose, microcrystalline cellulose, dibasic calcium phosphate, sucrose-based diluents, confectioner's sugar, monobasic calcium sulfate monohydrate, calcium sulfate dihydrate, calcium lactate trihydrate, dextrates, inositol, hydrolyzed cereal solids, amylose, powdered cellulose, calcium carbonate, glycine, or bentonite, and the like.
h) Anti-adherents or glidants such as talc, corn starch, DL-leucine, sodium lauryl sulfate, and magnesium, calcium, or sodium stearates, and the like.
i) Pharmaceutically compatible carriers such as acacia, gelatin, colloidal silicon dioxide, calcium glycerophosphate, calcium lactate, maltodextrin, glycerine, magnesium silicate, sodium caseinate, soy lecithin, sodium chloride, tricalcium phosphate, dipotassium phosphate, sodium stearoyl lactylate, carrageenan, monoglyceride, diglyceride, or pregelatinized starch, and the like.

The final formulation is preferably in the form of granules, compressed tablets, or capsules.

The formulation described above can be prepared using conventional formulation procedures and equipment. Therefore it is another aspect of the present invention to provide a method of preparing a formulation as described above comprising the following steps:

a) Mixing of the active substance of formula I with an alkaline compound or a mixture of alkaline compounds and optionally with one or more of the auxiliary materials;
b) Dissolving of the surfactant in a solvent, optionally with one or more of the auxiliary materials;
c) Addition of the solution comprising the surfactant in said solvent to the mixture containing the active substance and the alkaline compound, and optionally adding one or more auxiliary materials;
d) Drying and sieving of the granules obtained and optionally mixing with one or more auxiliary materials;
e) Optionally compressing of the mixture into tablets, optionally followed by coating, or filling the mixture into capsules.

In another embodiment of the invention, the formulation is prepared with a method comprising the following steps:

a) Dissolving the active substance of formula I in a solvent to give a first solution;
b) Dissolving of the surfactant in a solvent to give a second solution
c) Mixing of said first and second solution;
d) Co-precipitation of the active substance and the surfactant from the mixed solution by adding an anti-solvent
e) Mixing of the co-precipitate of the mixture containing the active substance and the surfactant with the alkaline compound, and optionally withone or more auxiliary materials;
f) Drying and sieving of the granules obtained and optionally mixing with one or more auxiliary materials;
g) Optionally compressing of the mixture into tablets, optionally followed by coating, or filling the mixture into capsules.

In an even further embodiment of the invention, the formulation is prepared with a method comprising the following steps:

a) Mixing of the active substance of formula I with an alkaline compound or a mixture of alkaline compounds with one or more surfactants and optionally with one or more of the auxiliary materials;
b) Compacting the mixture into compacts;
c) Breaking the compacts to form granules;
d) Mixing the granules with one or more auxiliary materials;
e) Optionally compressing of the mixture into tablets, optionally followed by coating, or filling the mixture into capsules.

When this dry formulation process described in paragraph [0021] is used with docusate as the surfactant, the docusate has to be comminuted. This comminution can be performed using the cryogenic milling technique as described above in par [0014]

Various steps may also be part of the process, such as drying, breaking, sieving, mixing and packaging, but these steps are no essential features in obtaining the formulation according to the present invention

When water is used as a solvent and the content of the active material is higher than 15% of the total weight of the formulation, the process comprising a compaction step is preferably used.

Solvents useful to dissolve the surfactant used in the present invention are e.g. dichloromethane, ethyl acetate, methyl t-butyl ether and water. The preferred solvent is water, preferably with a temperature of between 50 and 95 °C. The most preferred temperature when water is used as a solvent is 50 - 65 °C.

The following examples are only intended to further illustrate the invention, in more detail, and therefore these example are not deemed to restrict the scope of the invention in any way.

## EXAMPLES.

### Example 1. Materials and methods

<u>Materials.</u>

S-Ca can be prepared according to the prescription given in Examples 2 and 3 of WO03/059939 starting with the acid prepared according to Example 2 of EP 0733642.

Sodium bicarbonate can be obtained from Sigma Aldrich or Canton Labs, India.
Docusate sodium can be obtained from Sigma Aldrich.
All other auxiliary materials are readily commercially available.

Methods.

IN-VITRO DISSOLUTION TESTING

Dissolution system.

The dissolution of the tablets is determined with 900 ml of 0.05 mol/l phosphate buffer pH 6.8 as dissolution medium, using USP test-apparatus 2 (paddle) at 50 rpm. The quantity of dissolved S-H is determined by filtering the dissolution aliquots and after dilution analysing by UV absorbance at 240 nm. For external standardisation, 17.0 mg of Compound S-Ca is dissolved in 50 ml methanol, 2.0 ml of this solution with 2 ml dissolution medium diluted to 25 ml with methanol.

The quantity of dissolved Compound S-H, expressed in percent relative to the label claim, is given by the equation 1:

$$\%dissolved = \frac{Ab_T \times W_S \times 2 \times 900 \times 25 \times P}{Ab_S \times D_S \times 25 \times C \times 2 \times 100} \times 0.9638 \times 100$$

**Equation 1 Calculation of the quantity dissolved Compound S-H.**

Where,

$Ab_T$ = Absorbance of the test preparation.
Abs = Absorbance of the standard preparation.
Ws = Weight of Compound S-Ca standard taken (in mg).
Ds = Dilution for standard preparation.
P = Potency of Compound S-Ca standard (in percent).
C = Claim value of Compound S-H in each tablet (i.e. 150 mg or 300 mg)
0.9638 = Conversion factor for Compound S-Ca to Compound S-H.

**Example 2. Manufacturing of a formulation according to present invention with organic solvent granulation.**

PREPARATION OF THE FORMULATION

The quantities of S-Ca and sodium bicarbonate are sieved and mixed. Sodium docusate is dissolved in dichloromethane, and granulated with the blend to get well - kneaded dough. The granules are dried in a tray dryer. The dried granules are passed through a sieve, mixed with microcrystalline cellu lose, magnesium stearate, talc, and colloidal anhydrous silica, and compressed into tablets. The tablets are coated with Opadry suspended in organic solvents.

**Table 1. Composition of tablet containing 300 mg S-H prepared using non-aqeous method**

| Materials | Quantity/Tablet (mg) |
|---|---|
| Active substance (S-Ca)[*)] | 311.25 |
| Sodium bicarbonate | 600.00 |
| Docusate Na | 10.00 |
| Dichloromethane | 0.750 ml[1] |
| Microcrystalline Cellulose | 46.25 |
| Magnesium Stearate | 7.50 |
| Purified Talc | 7.50 |
| Colloidal Anhydrous Silica | 7.50 |

(continued)

| Materials | Quantity/Tablet (mg) |
|---|---|
| Film Coating Formula<br>Opadry White OY-IN-58091<br>2-Propanol<br>Dichloromethane | <br>30<br>0.165 ml[1]<br>0.330 ml[1] |
| *) calcium salt of 1H-1-Benzazepine-1-acetic acid, 3-[[[1-[(2R)-2-(ethoxycarbonyl)-4-phenylbutyl]cyclopentyl]carbonyl]amino]-2,3,4,5-tetrahydro-2-oxo-, (3S)-. (Compound S-Ca)<br>[1]Solvents are removed during formulation process | |

The dissolution curves of tablets manufactured according to this Example are given in Figure 1 (symbol ♦).

**Example 3. Manufacturing of a formulation according to present invention with aqueous granulation and compaction**

<u>PREPARATION OF THE FORMULATION</u>

About 33% of the required quantity of S-Ca and the required quantity of sodium hydrogen carbonate are mixed. The mixture is moistened with a hot aqueous solution of the required quantity of docusate sodium. The mixture is granulated, the granular material is dried and broken, and the remaining quantity of S-Ca and about 50% of the required quantities of sodium starch glycolate and magnesium stearate are mixed with the dried granulate, compacted, and broken.

The remaining quantities of sodium starch glycolate and magnesium stearate, and the required quantities of microcrystalline cellulose, talc and colloidal anhydrous silica are mixed with the granulate. The final granular material is compressed into tablets.

The tablets are coated by spraying an Opadry II suspension in water on the tablets.

**Table 2. Composition of the 300 mg tablet manufactured with aqueous granulation and compaction**

| materials | quantity/tablet (mg) |
|---|---|
| S-Ca | 311.25 |
| sodium bicarbonate | 600.0 |
| docusate sodium | 10.0 |
| microcryst. cellulose Avicel PH 101 | 36.25 |
| sodium starch glycolate type A | 20.0 |
| purified water | 36[1] |
| magnesium stearate | 7.5 |
| purified talc | 7.5 |
| colloidal silicon dioxide | 7.5 |
| Opadry II Yellow 85F22185 | 30 |
| [1]solvent removed during processing | |

The dissolution curves determined with the method described in Example 1 of tablets manufactured according to this Example are given in Figure 1 (symbol ■).

**Example 4. Manufacturing of a formulation according to present invention with aqueous granulation.**

<u>PREPARATION OF THE FORMULATION</u>

The required quantities of S-Ca, sodium hydrogen carbonate, about 50% of the amount of sodium starch glycolate and about 54% of the amount of microcrystalline cellulose are mixed. The mixture is moistened with a hot

aqueous solution of the required quantity of docusate sodium and Povidone K30). The mixture is granulated, the granular material is dried and broken.
The remaining quantities of sodium starch glycolate and microcrystalline cellulose, and the required quantities of magnesium stearate, talc and colloidal anhydrous silica are mixed with the dried granulate. The final granular material is compressed into tablets. The tablets are coated by spraying an Opadry II suspension in water on the tablets.

**Table 3. Composition of the 150 mg tablet manufactured with aqueous granulation**

| Materials | quantity/tablet (mg) |
|---|---|
| S-Ca | 155.63 |
| sodium bicarbonate | 600 |
| docusate sodium | 10.0 |
| microcryst. cellulose Avicel PH 101 | 161.87 |
| sodium starch glycolate type A | 40.0 |
| Povidone K30 | 10.0 |
| purified water | 72[1] |
| magnesium stearate | 7.5 |
| purified talc | 7.5 |
| colloidal silicon dioxide | 7.5 |
| Opadry II Yellow 85F22185 | 30 |
| [1]solvent removed during processing | |

The comparative dissolution curves of tablets manufactured according to Examples 3 and 4 are given in Figure 2 (symbol ♦ for 150 mg tablets and symbol ■ for 300 mg tablets).

**Example 5. Manufacturing of a standard 400 mg formulation**

PREPARATION OF THE FORMULATION

The required quantity of S-Ca is dry compacted in a roller compactor, broken and sieved. The required quantities of microcrystalline cellulose, cross-linked polyvinylpyrrolidone, and sodium stearyl fumarate are mixed with the compacted powder. The final granular material is compressed into tablets. The tablets are coated by spraying an Opadry II suspension in water on the tablets.

**Table 4. Composition of the 400 mg tablet manufactured without surfactant and alkaline material**

| Materials | quantity/tablet (mg) |
|---|---|
| S-Ca | 414.25 |
| microcryst, cellulose Avicel PH 301 | 249.0 |
| Kollidon CL | 14.0 |
| sodium stearyl fumarate | 1.75 |
| Opadry II Yellow 85F22185 | 21.0 |

**Example 6. Bio-availability study.**

In an open, randomized, cross-over, single dose study in healthy volunteers the bio-availability of the preferred formulation was compared with a formulation manufactured without carbonate and without surfactant. An oral solution is used as a reference. Compound S-Ca is used as the drug substance.
To the subjects the following formulations were administered:

- a solution containing an amount of active material corresponding to 200 mg compound S-H in citrate buffer
- a tablet formulation manufactured according to Example 1, with a composition as stated in Table 1.
- a tablet formulation prepared according to Example 5.

The bio-availability, dose-normalized, and calculated as the ratio to the solution formulation, are given in Table 5, and are illustrated in Figure 3.

**Table 5.** Bioavailability, measured as dose-normalized ratio of AUC over a citrate buffer formulation.

| formulation | ratio | Reference in Figure 3 |
|---|---|---|
| Citrate buffer formulation used as reference | 1.00 | A |
| tablet 300 mg, Example 1 | 1.07 | D |
| tablet 400 mg without surfactant, Example 5 | 0.81 | F |

## Claims

1. An oral immediate release formulation of an active compound of the general formula

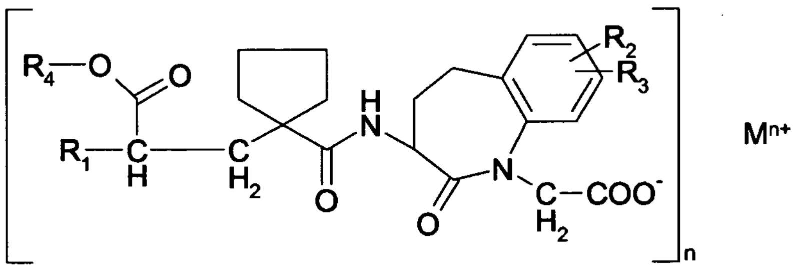

(I)

wherein:

$R_1$ is a selected from the group consistin g of $(C_1-C_6)$alkoxy$(C_1-C_6)$alkyl which may be substituted by a $(C_1-C_6)$ alkoxy, phenyl-$(C_1-C_6)$-alkyl and phenyloxy-$(C_1-C_6)$-alkyl wherein the phenylgroup may be substituted with $(C_1-C_6)$alkyl, $(C_1-C_6)$alkoxy or halogen, and naphtyl-$(C_1-C_6)$-alkyl,
$R_2$ and $R_3$ are both independently hydrogen or halogen,
$R_4$ is a biolabile ester forming group,
M is a hydrogen or a metal ion, preferably a bivalent metal ion.
n is 1, 2 or 3;

comprising

a) said active substance in an amount of up to 65% of the total weight of the formulation;
b) at least 10% w/w an alkaline compound or a mixture of alkaline compounds;
c) between 0.1 and 10% w/w of one or more surfactants, and
d) optionally comprises auxiliary materials in an amount of between 1% and 45% of the total weight of the formulation.

2. An oral immediate release formulation according to claim 1, wherein the alkaline compound is selected from the group consisting of inorganic and organic alkaline compounds, such as sodium bicarbonate, potassium bicarbonate, sodium carbonate, potassium carbonate, sodium citrate, tris buffer, triethanolamine, alkaline hydroxides such as sodium hydroxide, potassium hydroxide or magnesium hydroxide, alkaline phosphates such as dipotassium hydrogen phosphate, and meglumine or mixtures of these alkaline compounds.

**3.** An oral immediate release formulation according to claim 1 or 2, wherein the surfactant is a hydrophilic surfactant.

**4.** An oral immediate release formulation according to claim 3, wherein the hydrophilic surfactant is selected from the group comp rising of cremophores, poloxamers, polyoxyethylene sorbitan esters, docusate and pharmaceutically acceptable docusate salts, or mixtures thereof.

**5.** An oral immediate release formulation according to claim 4, wherein the surfactant is selected from the group consisting of docusate sodium, docusate potassium, docusate calcium.

**6.** An oral immediate release formulation according to claims 1 -5, wherein M is calcium in its 2+ form.

**7.** An oral immediate release formulation according to claims 1-6, wherein the weight ratio between the surfactant and the active substance is between 1:200 and 1:5.

**8.** An oral immediate release formulation according to claims 1 -7, wherein the weight ratio between the active substance and the alkaline compound is between 1:6 and 1:0.5.

**9.** An oral immediate release formulation according to claim 1-8, **characterised in that** the amount of alkaline compound is more than 55% w/w, preferably more than 60% w/w.

**10.** An oral immediate release formulation according to claims 1 -9, **characterized in that** the alkaline compound is sodium bicarbonate.

**11.** An oral immediate release formulation according to claims 1-10, **characterized in that** the surfactant ingredient is docusate sodium.

**12.** An oral immediate release formulation according to claims 1-11, **characterized in that** said active substance is the calcium salt of 1H-1-Benzazepine-1-acetic acid, 3-[Q1-[2-(ethoxycarbonyl)-4-phenylbutyl]-cyclopentyl]carbonyl]-amino]-2,3,4,5-tetrahydro-2-oxo-, preferably in its 3S,2'R form.

**13.** An oral immediate release formulation according to claims 1-12 in the form of granules, compressed tablets or capsules.

**14.** A method of preparing a formulation according to claims 1-13, comprising the following steps:

a) Mixing of the active substance of formula I with an alkaline compound or a mixture of alkaline compounds and optionally with one or more of the auxiliary materials;
b) Dissolving of the surfactant in a solvent, optionally with one or more of the auxiliary materials;
c) Addition of the solution comprising the surfactant in said solvent to the mixture containing the active substance and the alkaline compound, and optionally adding one or more auxiliary materials;
d) Drying and sieving of the granules obtained and optionally mixing with one or more auxiliary materials;
e) Optionally compressing of the mixture into tablets, optionally followed by coating or filling the mixture into capsules.

**15.** A method of preparing a formulation according to claims 1 -13, comprising the following steps:

a) Dissolving the active substance of formula I in a solvent to give a first solution;
b) Dissolving of the surfactant in a solvent to give a second solution
c) Mixing of said first and second solution;
d) Co-precipitation of the active substance and the surfactant from the mixed solution by adding an anti-solvent
e) Mixing of the co-precipitate of the mixture containing the active substance and the surfactant with the alkaline compound, and optionally withone or more auxiliary materials;
f) Drying and sieving of the granules obtained and optionally mixing with one or more auxiliary materials;
g) Optionally compressing of the mixture into tablets, optionally followed by coating, or filling the mixture into capsules.

**16.** A method of preparing a formulation according to claims 1 -13, comprising the following steps:

a) Mixing of the active substance of formula I with an alkaline compound or a mixture of alkaline compounds with one or more surfactants and optionally with one or more of the auxiliary materials;
b) Compacting the mixture into compacts;
c) Breaking the compacts to form granules;
d) Mixing the granules with one or more auxiliary materials;
e) Optionally compressing of the mixture into tablets, optionally followed by coating or filling the mixture into capsules.

**17.** The method according to claim 16, wherein the surfactant is docusate and wherein the docusate added is subjected to comminution by cryogenic milling before the mixing step.

**Patentansprüche**

**1.** Orale Formulierung zur sofortigen Freigabe eines Wirkstoffs der allgemeinen Formel

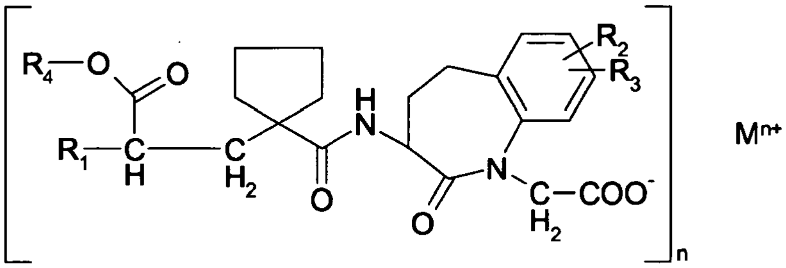

**(I)**

worin:

$R_1$ ausgewählt ist aus der Gruppe bestehend aus $(C_1\text{-}C_6)$-Alkoxy- $(C_1\text{-}C_6)$ -alkyl, welche substituiert sein kann durch ein $(C_1\text{-}C_6)$-Alkoxy, Phenyl-$(C_1\text{-}C_6)$ -alkyl und Phenyloxy-$(C_1\text{-}C_6)$-alkyl, worin die Phenylgruppe substituiert sein kann mit $(C_1\text{-}C_6)$-Alkyl, $(C_1\text{-}C_6)$-Alkoxy oder Halogen, und Naphthyl-$(C_1\text{-}C_6)$-alkyl,
$R_2$ und $R_3$ beide unabhängig Wasserstoff oder Halogen darstellen,
$R_4$ eine biolabilen Ester bildende Gruppe darstellt,
M für ein Wasserstoff oder ein Metallion, vorzugsweise ein zweiwertiges Metallion steht,
n 1, 2 oder 3 ist;

welche umfasst:

a) besagten Wirkstoff in einer Menge von bis zu 65% des Gesamtgewichts der Formulierung;
b) mindestens 10% Gew./Gew.einer alkalischen Verbindung oder eines Gemisches aus alkalischen Verbindungen;
c) zwischen 0,1 und 10% Gew./Gew.von einem oder mehreren oberflächenaktiven Stoffen und
d) gegebenenfalls Hilfsstoffe in einer Menge von zwischen 1% und 45% des Gesamtgewichts der Formulierung umfasst.

**2.** Orale Formulierung zur sofortigen Freigabe gemäß Anspruch 1, worin die alkalische Verbindung ausgewählt ist aus der Gruppe bestehend aus anorganischen und organischen alkalischen Verbindungen wie Natriumbicarbonat, Kaliumbicarbonat, Natriumcarbonat, Kaliumcarbonat, Natriumcitrat, Trispuffer, Triethanolamin, alkalischen Hydroxiden wie Natriumhydroxid, Kaliumhydroxid oder Magnesiumhydroxid, alkalischen Phosphaten wie Dikaliumhydrogenphosphat und Meglumin oder Gemischen aus diesen alkalischen Verbindungen.

**3.** Orale Formulierung zur sofortigen Freigabe gemäß Anspruch 1 oder 2, worin der oberflächenaktive Stoff ein hydrophiler oberflächenaktiver Stoff ist.

**4.** Orale Formulierung zur sofortigen Freigabe gemäß Anspruch 3, worin der hydrophile oberflächenaktive Stoff ausgewählt ist aus der Gruppe umfassend Cremophore, Poloxamere, Polyoxyethylensorbitanester, Docusat und pharmazeutisch annehmbare Docusatsalze oder Gemische daraus.

**5.** Orale Formulierung zur sofortigen Freigabe gemäß Anspruch 4, worin der oberflächenaktive Stoff ausgewählt ist aus der Gruppe bestehend aus Docusatnatrium, Docusatkalium, Docusatcalcium.

**6.** Orale Formulierung zur sofortigen Freigabe gemäß Ansprüchen 1-5, worin M für Calcium in seiner 2+ Form steht.

**7.** Orale Formulierung zur sofortigen Freigabe gemäß Ansprüchen 1-6, worin das Gewichtsverhältnis zwischen dem oberflächenaktiven Stoff und dem Wirkstoff zwischen 1:200 und 1:5 ist.

**8.** Orale Formulierung zur sofortigen Freigabe gemäß Ansprüchen 1-7, worin das Gewichtsverhältnis zwischen dem Wirkstoff und der alkalischen Verbindung zwischen 1:6 und 1:0,5 ist.

**9.** Orale Formulierung zur sofortigen Freigabe gemäß Anspruch 1-8, **dadurch gekennzeichnet, dass** die Menge an alkalischer Verbindung mehr als 55% Gew./Gew., vorzugsweise mehr als 60% Gew./Gew. ist.

**10.** Orale Formulierung zur sofortigen Freigabe gemäß Ansprüchen 1-9, **dadurch gekennzeichnet, dass** die alkalische Verbindung Natriumbicarbonat ist.

**11.** Orale Formulierung zur sofortigen Freigabe gemäß Ansprüchen 1-10, **dadurch gekennzeichnet, dass** der oberflächenaktive Inhaltsstoff Docusatnatrium ist.

**12.** Orale Formulierung zur sofortigen Freigabe gemäß Ansprüchen 1-11, **dadurch gekennzeichnet, dass** besagter Wirkstoff das Calciumsalz von 1H-1-Benzazepin-1-essigsäure, 3-[[[1-[2-(Ethoxycarbonyl)-4-phenylbutyl]-CyClopentyl]carbonyl]-amino]-2,3,4,5-tetrahydro-2-oxo-, vorzugsweise in seiner 3S,2'R-Form ist.

**13.** Orale Formulierung zur sofortigen Freigabe gemäß Ansprüchen 1-12 in der Form von Körnern, komprimierten Tabletten oder Kapseln.

**14.** Verfahren zum Herstellen einer Formulierung gemäß Ansprüchen 1-13, welches die folgenden Schritte umfasst:

a) Mischen des Wirkstoffs der Formel I mit einer alkalischen Verbindung oder einem Gemisch aus alkalischen Verbindungen und gegebenenfalls mit einem oder mehreren der Hilfsstoffe;
b) Lösen des oberflächenaktiven Stoffs in einem Lösungsmittel, gegebenenfalls mit einem oder mehreren der Hilfsstoffe;
c) Zugabe der den oberflächenaktiven Stoff in besagtem Lösungsmittel umfassenden Lösung zum Gemisch, welches den Wirkstoff und die alkalische Verbindung umfasst, und gegebenenfalls Zugeben von einem oder mehreren Hilfsstoffen;
d) Trocknen und Sieben der erhaltenen Körner und gegebenenfalls Mischen mit einem oder mehreren Hilfsstoffen;
e) Gegebenenfalls Komprimieren des Gemisches in Tabletten, gegebenenfalls gefolgt von Überziehen oder Füllen des Gemisches in Kapseln.

**15.** Verfahren zum Herstellen einer Formulierung gemäß Ansprüchen 1-13, welches die folgenden Schritte umfasst:

a) Lösen des Wirkstoffs der Formel I in einem Lösungsmittel, um eine erste Lösung zu ergeben;
b) Lösen des oberflächenaktiven Stoffs in einem Lösungsmittel, um eine zweite Lösung zu ergeben
c) Mischen der besagten ersten und zweiten Lösung;
d) Co-Präzipitation des Wirkstoffs und des oberflächenaktiven Stoffs aus der gemischten Lösung durch Zugeben eines Anti-Lösungsmittels
e) Mischen des Co-Präzipitats des Gemisches, welches den Wirkstoff und den oberflächenaktiven Stoff enthält, mit der alkalischen Verbindung, und gegebenenfalls mit einem oder mehreren Hilfsstoffen;
f) Trocknen und Sieben der erhaltenen Körner und gegebenenfalls Mischen mit einem oder mehreren Hilfsstoffen;
g) gegebenenfalls Komprimieren des Gemisches in Tabletten, gegebenenfalls gefolgt von Überziehen, oder Füllen des Gemisches in Kapseln.

16. Verfahren zum Herstellen einer Formulierung gemäß Ansprüchen 1-13, welches die folgenden Schritte umfasst:

a) Mischen des Wirkstoffs der Formel I mit einer alkalischen Verbindung oder einem Gemisch aus alkalischen Verbindungen mit einem oder mehreren oberflächenaktiven Stoffen und gegebenenfalls mit einem oder mehreren der Hilfsstoffe;
b) Kompaktieren des Gemisches in Kompakte;
c) Brechen der Kompakte, um Körner zu bilden;
d) Mischen der Körner mit einem oder mehreren Hilfsstoffen;
e) Gegebenenfalls Komprimieren des Gemisches in Tabletten, gegebenenfalls gefolgt von Überziehen oder Füllen des Gemisches in Kapseln.

17. Verfahren gemäß Anspruch 16, wobei der oberflächenaktive Stoff Docusat ist und wobei das zugegebene Docusat Zerkleinerung durch kryogenisches Mahlen vor dem Mischschritt unterworfen wird.

## Revendications

1. Formulation à libération immédiate pour administration orale d'un composé actif de la formule générale

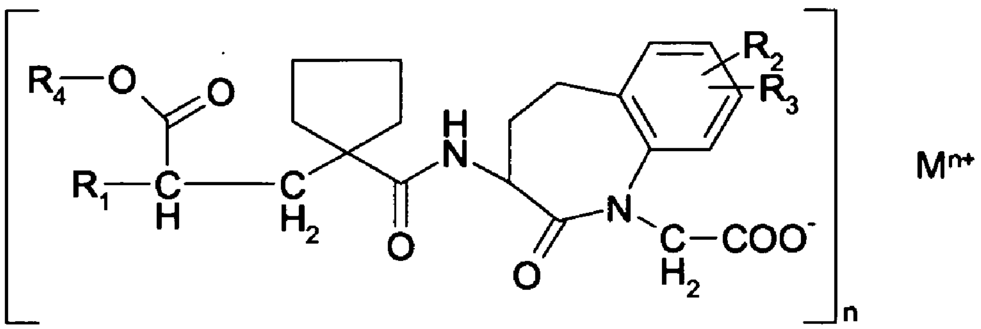

(I)

dans laquelle :

$R_1$ est choisi dans le groupe constitué du groupe ($C_1$-$C_6$)alcoxy($C_1$-$C_6$)alkyle qui peut être substitué par un groupe ($C_1$-$C_6$)alcoxy, phényl-($C_1$-$C_6$)alkyle et phényloxy-($C_1$-$C_6$)alkyle, où le groupe phényle peut être substitué par un groupe ($C_1$-$C_6$)alkyle, ($C_1$-$C_6$)alcoxy ou un halogène, et du groupe napthyl-($C_1$-$C_6$)-alkyle,
$R_2$ et $R_3$ sont tous deux indépendamment l'hydrogène ou un halogène,
$R_4$ est un groupe formant un ester bio-instable,
M est un hydrogène ou un ion métallique, de préférence un ion métallique bivalent,
n est égal à 1, 2 ou 3 ;

comprenant

a) ladite substance active dans une quantité jusqu'à 65 % du poids total de la formulation ;
b) au moins 10 % en poids/poids d'un composé alcalin ou d'un mélange de composés alcalins ;
c) de 0,1 à 10 % en poids/poids d'un ou plusieurs tensioactifs ; et
d) comprend éventuellement des matières auxiliaires dans une quantité de 1 % à 45 % du poids total de la formulation.

2. Formulation à libération immédiate pour administration orale selon la revendication 1, dans laquelle le composé alcalin est choisi dans le groupe constitué de composés alcalins inorganiques et organiques, tels que le bicarbonate de sodium, le bicarbonate de potassium, le carbonate de sodium, le carbonate de potassium, le citrate de sodium, un tampon tris, la triéthanolamine, des hydroxydes alcalins, tels que l'hydroxyde de sodium, l'hydroxyde de potassium ou l'hydroxyde de magnésium, des phosphates alcalins, tels que l'hydrogénophosphate de dipotassium et la méglumine ou des mélanges de ces composés alcalins.

3. Formulation à libération immédiate pour administration orale selon la revendication 1 ou 2, dans laquelle le tensioactif est un tensioactif hydrophile.

4. Formulation à libération immédiate pour administration orale selon la revendication 3, dans laquelle le tensioactif hydrophile est choisi dans le groupe comprenant des crémophores, des poloxamères, des esters de polyoxyéthylène sorbitan, des docusates et des sels de docusate pharmaceutiquement acceptables ou des mélanges de ceux-ci.

5. Formulation à libération immédiate pour administration orale selon la revendication 4, dans laquelle le tensioactif est choisi dans le groupe constitué du docusate de sodium, du docusate de potassium, du docusate de calcium.

6. Formulation à libération immédiate pour administration orale selon les revendications 1-5, dans laquelle M est le calcium dans sa forme 2+.

7. Formulation à libération immédiate pour administration orale selon les revendications 1-6, dans laquelle le rapport massique entre le tensioactif et la substance active est de 1:200 à 1:5.

8. Formulation à libération immédiate pour administration orale selon les revendications 1-7, dans laquelle le rapport massique entre la substance active et le composé alcalin est de 1:6 à 1:0,5.

9. Formulation à libération immédiate pour administration orale selon les revendications 1-8, **caractérisée en ce que** la quantité de composé alcalin est supérieure à 55 % en poids/poids, encore mieux à 60 % poids/poids.

10. Formulation à libération immédiate pour administration orale selon les revendications 1-9, **caractérisée en ce que** le composé alcalin est le bicarbonate de sodium.

11. Formulation à libération immédiate pour administration orale selon les revendications 1-10, **caractérisée en ce que** l'ingrédient tensioactif est le docusate de sodium.

12. Formulation à libération immédiate pour administration orale selon les revendications 1-11, **caractérisée en ce que** ladite substance active est le 1H-1-benzazépine-1-acétate de calcium, le 3-[[[1-[2-(éthoxycarbonyl)-4-phénylbutyl]cyclopentyl]carbonyl]-amino]-2,3,4,5-tétrahydro-2-oxo-, de préférence dans sa forme 3S,2'R.

13. Formulation à libération immédiate pour administration orale selon les revendications 1-12 dans la forme de granulés, de tablettes comprimées ou de capsules.

14. Procédé de préparation d'une formulation selon les revendications 1-13, comprenant les étapes suivantes consistant :

   a) à mélanger la substance active de la formule (I) avec un composé alcalin ou un mélange de composés alcalins et éventuellement avec un ou plusieurs des matériaux auxiliaires ;
   b) à dissoudre le tensioactif dans un solvant, éventuellement avec un ou plusieurs des matériaux auxiliaires ;
   c) à ajouter la solution comprenant le tensioactif dans ledit solvant au mélange contenant la substance active et le composé alcalin, et à ajouter éventuellement un ou plusieurs matériaux auxiliaires ;
   d) à sécher et à tamiser les granulés obtenus et à les mélanger éventuellement avec un ou plusieurs matériaux auxiliaires ;
   e) à comprimer éventuellement le mélange en tablettes, éventuellement suivi par le revêtement ou le remplissage du mélange dans des capsules.

15. Procédé de préparation d'une formulation selon les revendications 1-13 comprenant les étapes suivantes consistant :

   a) à dissoudre la substance active de la formule (I) dans un solvant pour fournir une première solution ;
   b) à dissoudre le tensioactif dans un solvant pour fournir une seconde solution ;
   c) à mélanger lesdites première et seconde solutions ;
   d) à faire co-précipiter la substance active et le tensioactif de la solution mélangée en ajoutant un anti-solvant ;
   e) à mélanger le co-précipité du mélange contenant la substance active et le tensioactif avec le composé alcalin, et éventuellement avec un ou plusieurs matériaux auxiliaires ;

f) à sécher et à tamiser les granulés obtenus et à mélanger éventuellement avec un ou plusieurs matériaux auxiliaires ;
g) à comprimer éventuellement le mélange en tablettes, éventuellement suivi par un revêtement ou un remplissage du mélange dans des capsules.

**16.** Procédé de préparation d'une formulation selon les revendications 1-13 comprenant les étapes suivantes consistant :

a) à mélanger la substance active de la formule (I) avec un composé alcalin ou un mélange de composés alcalins avec un ou plusieurs tensioactifs et éventuellement avec un ou plusieurs des matériaux auxiliaires ;
b) à compacter le mélange en produits compactés ;
c) à casser les produits compactés pour former des granulés ;
d) à mélanger les granulés avec un ou plusieurs matériaux auxiliaires ;
e) à comprimer éventuellement le mélange en comprimés, éventuellement suivi par un revêtement ou un remplissage du mélange dans des capsules.

**17.** Procédé selon la revendication 16, dans lequel le tensioactif est un docusate et dans lequel le docusate ajouté est soumis à un broyage par broyage cryogénique avant l'étape de mélange.

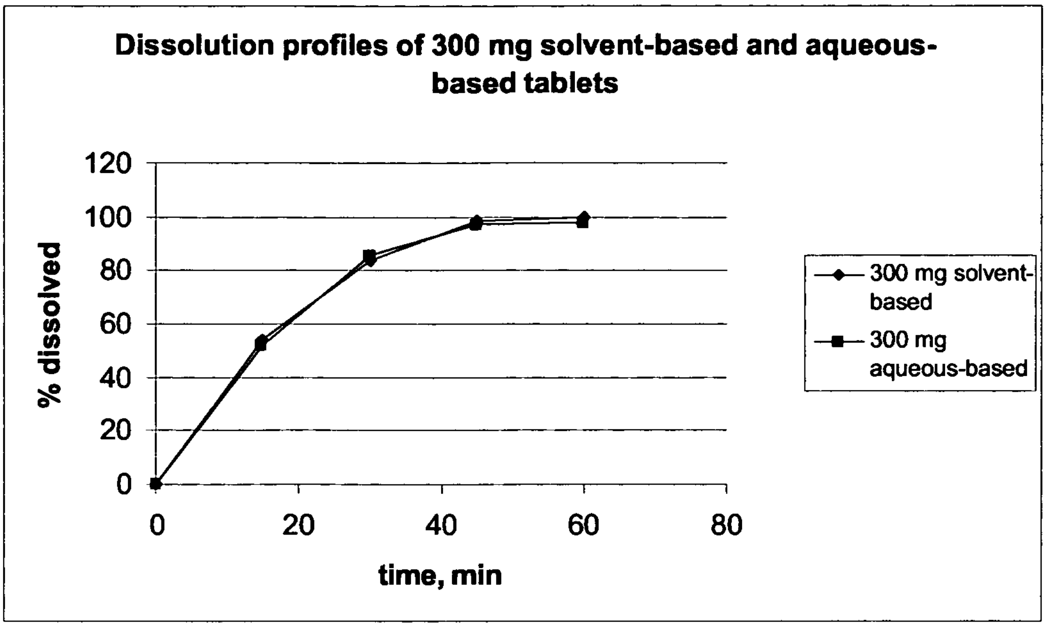


**Figure 1.** **Comparative dissolution profiles of tablets containing 300 mg S-H made by solvent and aqueous process**

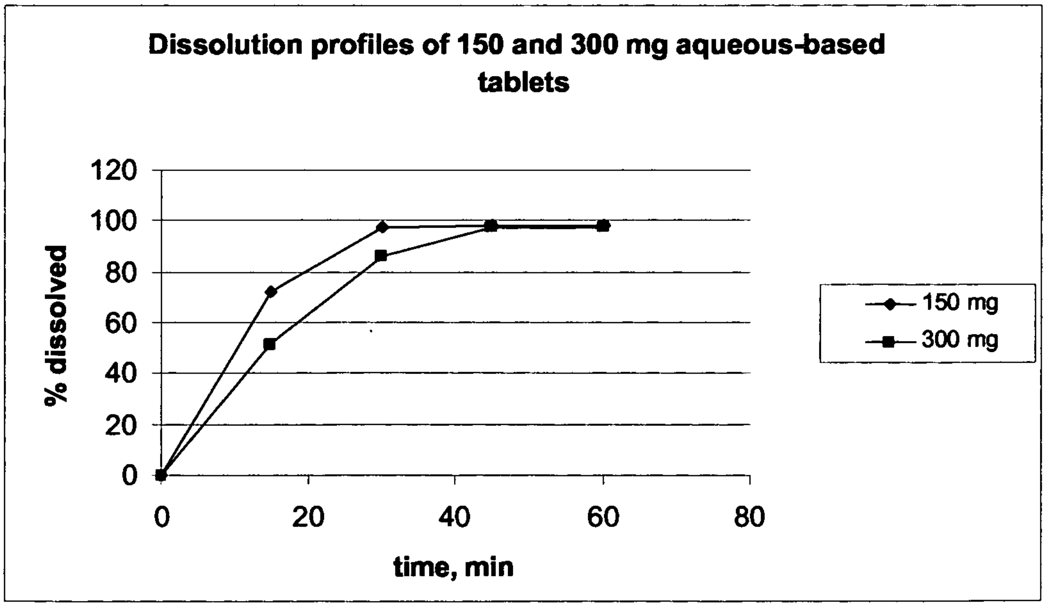


**Figure 2.** **Comparative dissolution profiles of tablets containing 150 or 300 mg S-H made by the aqueous process**

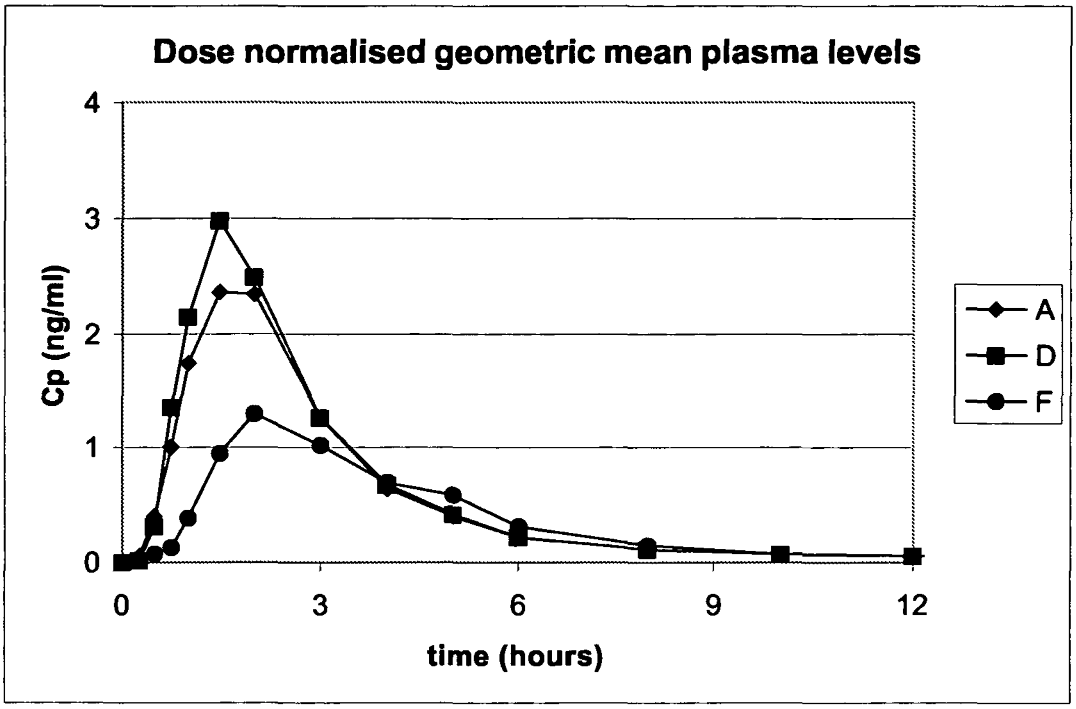


**Figure 3. Comparative, dose-normalized pharmacokinetic profiles showing plasma concentrations of the active metabolite of compound S -H, of example formulations:**

**A: citrate buffer formulation used as a reference**
**D: tablet 300 mg, Example 1**
**F: tablet 400 mg without surfac tant, Example 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0000179 A
- WO 03068266 A
- EP 0733642 A
- WO 03059939 A